# EUROPEAN PATENT APPLICATION

(11) **EP 2 997 951 A1**
(43) Date of publication of application: **23.03.2016**
(21) Application number: 13872272.3
(22) Date of filing: 29.10.2013
(51) Int. Cl.: A61H 1/02

(54) **JOINT REHABILITATION TRAINING SYSTEM BASED ON REMOTE CONTROL AND IMPLEMENTATION METHOD THEREFOR**

(30) Priority: 31.05.2013 CN 201310212516
(71) Applicant: Sichuan Xukang Medical Electrical Equipment Co., Ltd., Chengdu, Sichuan 611730 (CN)
(72) Inventor: LI, Tong, Chengdu Sichuan 610000 (CN)
(74) Representative: Cabinet Chaillot
(86) International application number: PCT/CN2013/001304
(87) International publication number: WO 2014/190459

(57) **Abstract**

A joint rehabilitation training system, comprising:the training protector and the angle adjustment device set on the training protector, as well as the telemedicine terminal, the control terminal with wireless connection to the telemedicine terminal, and the angle measuring device set on the angle adjustment device used for measuring the training angle of the training protector. The control terminal includes the central processing unit connected to the angle measuring device, the wireless communication module connected to the central processing unit with wireless communication with the telemedicine terminal, and the display and the speaker respectively connected to the central processing unit. This invention has reasonable design. The training angle has high adjustment precision. It is easy to use. The training effect is good. It is suitable for popularization and application.

## Description

### Field of the Invention

This invention relates to a rehabilitation training system. Specifically, it involves a joint rehabilitation training system based on the remote control and its implementation method.

### Background of the Invention

At present, the exercise rehabilitation training device for joint (such as elbow, finger, wrist or knee joint) is generally made up of the training protector. Its structure usually includes the upper bracket, the lower bracket and the angle adjustment device. The upper bracket and the lower bracket are fixed on the patient's joint. The patient uses the angle adjustment device to adjust the angle between the two brackets according to the training plan, so that the joint of the patient is fixed on this angle. And then the patient can maintain this angle for the rehabilitation training.

However, the existing training protectors cannot realize the interaction between the doctor and the patient. It means the equipment is unable to provide the doctor with the patient's current training effect, training compliance and body load capacity in the process of the training. The patient cannot get the latest training plan from the doctor. In the process of training, the deviation between the training angle and the actual function may be too big. In the process of training, the body and the joint may be unable to bear the training load. In case of this kind of problem, the rehabilitation training will not have the predetermined effect. Instead it will cause harm to the patient.

### Summary of the invention

The purpose of this invention is to provide a joint rehabilitation training system based on the remote control and its implementation method, mainly to solve the problem of the interaction between doctor and patient which cannot be realized by the existing joint rehabilitation training device, and the resulting decline of the effect of rehabilitation training.

In order to achieve the above purpose, this invention adopts the following technical proposals:
The joint rehabilitation training system based on the remote control includes the training protector and the angle adjustment device set on the training protector, as well as the telemedicine terminal, the control terminal with wireless connection to the telemedicine terminal, and the angle measuring device set on the angle adjustment device used for measuring the training angle of the training protector.

The control terminal includes the central processing unit connected to the angle measuring device, the wireless communication module connected to the central processing unit with wireless communication with the telemedicine terminal, and the display and the speaker respectively connected to the central processing unit. And on the training protector, the physiological parameter detecting device connected to the central processing unit is set.

Between the training protector and the angle adjustment device, the first lock switch is set. At the same time on the training protector the protector length adjusting mechanism for the adjustment of the length of protector is set.

Further, this invention provides the following two solutions:
I. The training protector includes the base, the mounting plate set on the upper end of the base, and the supporting plate installed on the mounting plate. The angle adjustment device is installed between the mounting plate and the base; the protector length adjusting mechanism includes the slide set on the mounting plate, and the second lock switch set on the supporting plate matching with the slide used for the positioning of the supporting plate.
II. The training protector includes the base, the mounting plate set on the upper end of the base, and the supporting plate installed on the mounting plate. The angle adjustment device is installed between the mounting plate and the base; the protector length adjusting mechanism includes the main location hole set on the mounting plate, the accessory location hole set on the supporting plate, and the locating pin used to lock the main location hole and accessory location hole.

On the basis of the above two solutions, the angle measuring device is the absolute value encoder or the angular displacement sensor. The physiological parameter detecting device includes the temperature sensor, the blood pressure measuring cuff, the pressure sensor, the electrode slice and the ultrasonic probe respectively set on the training protector connected to the central processing unit.

Accordingly, this invention provides the implementation method of the joint rehabilitation training system based on the remote control, including the following steps:
(1) The patient fixes the training protector on the joint to be trained;
(2)The telemedicine terminal makes the initial training plan, and sends it to the control terminal;
(3) According to the training plan, the patient uses the angle adjustment device to adjust the training angle of the training protector, and adjusts the length of the training protector through the length adjusting mechanism of the training protector, so that it is consistent with the training plan;
(4) The training protector is started and the patient begins the rehabilitation training;
(5) During the training, the patient's current physical data is detected with the physiological parameter detecting device and transmitted to the control terminal or the telemedicine terminal. The human body's physiological load and joint/bone tolerance in the training process are monitored in real time. It automatically prompts whether the training intensity is moderate, and ensures the training safety.
(6) After completion of a rehabilitation training, through the physiological parameter detecting device the patient's current physical data is detected and transmitted to the control terminal or the telemedicine terminal, to obtain the new training plan;
(7) Step (3)~(6) are repeated until the patient recovers fully.

Further, Step (3) includes the following steps:
(3a) The wireless communication module receives the training plan and transmits it to the central processing unit;
(3b) The control display of the central processing unit displays the content of the training plan;
(3c) The patient adjusts the training angle of the training protector according to the angle displayed using the angle adjustment device, and adjusts the total length of the mounting plate and the supporting plate according to the length of the training part;
(3d) The angle measuring device measures the training angle of the training protector in real time and feedbacks to the central processing unit;
(3e) The control display of the central processing unit displays the current angle of the training protector. The patient modifies the angle of the training protector according to the current angle of the training protector displayed on the monitor, so that it is consistent with the angle set in the training plan.

Further, in Step (3a), the wireless communication module transmits the training plan to the central processing unit and automatically stores.

Furthermore, in Step (4), when the patient begins the rehabilitation training, the physiological parameter detecting device also begins to collect the physiological parameters of the patient, and transmits to the telemedicine terminal through the wireless communication module. The telemedicine terminal diagnoses the current state of rehabilitation training of the patient in real time.

In addition, based on the above hardware structure, this invention also provides the evaluation method of joint range of motion in the corresponding joint rehabilitation training, including the following steps:
(a) Adjust the total length of the mounting plate and the supporting plate to the length suitable for wearing according to the length of limb;
(b) Fix the training protector to the joint requiring the rehabilitation training;
(c) The telemedicine terminal makes the evaluation plan, and sends it to the control terminal;
(d) Turn on the lock switch on the training protector. Actively bend and stretch the target join according to the evaluation plan displayed by the control terminal. Drive the rotation of the training protector. The angle to be reached is sent by the angle measuring device to the telemedicine terminal;
(f) The telemedicine terminal judges the joint range of motion of the patient based on the current patient physiological parameters of the patient and the angle value measured by the angle measuring device.

Compared with the existing technology, this invention has the following beneficial effects:
(1) This invention has reasonable design, convenient operation and easy manufacturing.
(2) This invention uses the combination of the telemedicine terminal, the control terminal and the protector to realize the purpose of remote control of the patient's joint rehabilitation training. The training plan is made by the telemedicine terminal, and then transmitted to the control terminal. The control terminal adjusts the training angle of the training protector according to training plan. The patient can complete the joint rehabilitation training according to the angle in the training plan in the planned period. This invention has high degree of automation, realizes the intelligent rehabilitation training effect, greatly improves the training effect, and avoids the wrong operation caused by manual operation of the training device. The remote control technology is more and more mature today. This invention follows the trend of the development of science and technology, integrates the remote control technology into the rehabilitation training system, and realizes the automation operation of the rehabilitation training, so that the medical rehabilitation training technology gets the new progress on the research direction. It is a big leap in the field of the rehabilitation training.
(3) This invention uses the remote terminal to automatically control the rehabilitation training device. It can adjust the training angle in strict accordance with the training plan and guarantee the accuracy, and it does not need assistance from medical personnel or operation of the patient or others. It saves the manpower, and the operation is simple and convenient. At the same time, by moving the rehabilitation training protector, the rehabilitation training can be completed anytime and anywhere, completely avoiding the limitation of time and place. Therefore, compared with the existing technology, this invention has prominent substantive feature and notable progress.
(4) This invention uses the absolute value encoder or the angular displacement sensor to measure the training angle of the training protector. It can convert the angular displacement or linear displacement into the electrical signal, and then transfer to the central controller for processing and displaying. Because the absolute value encoder and the angular displacement sensor are characterized by no need of memory or searching of reference points. It does not have to always count. Whenever you need to know the position, you can go to read it. Therefore, the absolute value encoder and the angular displacement sensor set in this invention can allow the patient to know the training angle and training conditions of the current training protector accurately at any time.
(4) The wireless communication module in this invention transmits the training plan to the central processing unit and automatically stores, so as to call directly when needed. There is no need to access to the training plan through the network, so as to avoid the impact on the process of rehabilitation training due to limitation of the network. The rehabilitation training is based on the network. At the same time it can break away from the network to a certain extent, which provides the effective guarantee for the smooth development of the rehabilitation training.
(5) Tis invention also has a physiological parameter detecting device. In the process of training of the patient, it can collect the patient's physiological parameters in real time and transmit these physiological parameters to the telemedicine terminal, allowing doctors to know the current conditions of the patient comprehensively in real time, so as to timely adjust the training plan, form a circulating reparative rehabilitation training process of setting-implementation-inspection-fixing-implementation-inspection-revising. Thus the patient is always in the most appropriate training state, until he/she fully recovers, so as to avoid the poor repeated effect and even useless rehabilitation training.
(6) The first lock switch set in this invention can control the state between the mounting plate and the angle adjustment device, so that the mounting plate can rotate with the angle adjustment device or rotate separately. Thus, this device has wider angle adjusting range and higher controlling accuracy when used. It is closer to the training plan, so as to improve the effect of training, avoid the reduction of training effect due to the angle error and the possible training injury.
(7) The length adjusting mechanism of protector set in this invention can flexibly change the relative position between the mounting plate and the supporting plate, so as to change the length of the whole training protector and adapt to the joint length of different groups, which improves the scope of application of this technology and ensures the training effect.
(8) This invention is cost-effective and targeted. The training method is novel. It has a broad market application prospect and huge development potential. Therefore, this invention has high practical value and popularization value.

### Brief Description of the Drawings

Figure 1 is the schematic diagram of the structure of this invention.
Figure 2 is the schematic diagram of the structure of joint rehabilitation training system of the elbow joint in the implementation example of this invention.
Figure 3 is the schematic diagram of the structure of joint rehabilitation training system of the leg joint in the implementation example of this invention.

Among them, the names of parts corresponding to the reference number are as follows:
1-telemedicine terminal, 2-wireless communication module, 3-central processing unit, 4-monitor, 5-speaker, 6-training protector, 7-angle measuring device, 8-temperature sensor, 9-blood pressure measuring cuff, 10-pressure sensor, 11-electrode slice, 12-ultrasound probe, 13-the first lock switch, 14-base, 15-mounting plate, 16-supporting plate, 17-slide, 18-accessory location hole, 19-the second lock switch.

**Detailed Description of the Preferred Embodiments** Next, combined with the appended drawings and implementation examples, we will further describe this invention. The implementation methods of this invention include but not limited to the following implementation examples.

### Implementation example

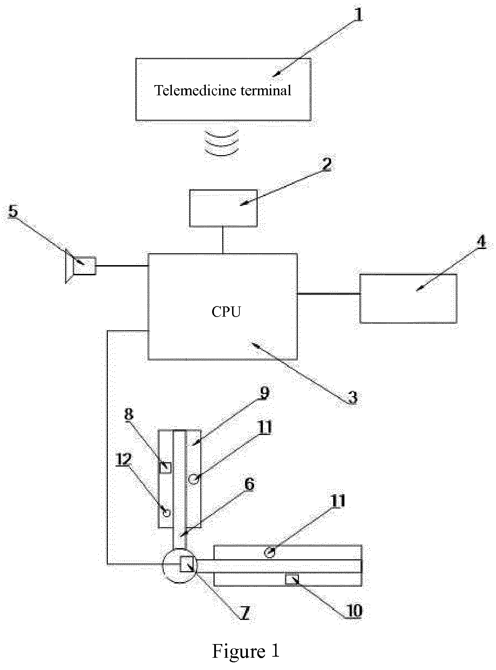

As shown in Figure 1~3, this invention includes the telemedicine terminal, the control terminal, the training protector, the angle measuring device and the physiological parameter detecting device. The training protector mainly includes the base, the angle adjustment device installed on the upper end of the base, the mounting plate connected to the angle adjustment device and the supporting plate installed on the mounting plate. In this implementation example, the angle measuring device is the absolute value encoder or the angular displacement sensor. It is fixed on the angle adjustment device of the training protector for the real-time measuring of the angle of the mounting plate. When the angle measuring device is the absolute value encoder, the model can be E6CP-AG5C-C, HN3806A5V4096, TRD-NA360NWF or ROTARY ENCODER SSI. The manufacturer is Omron Corporation, Hangning Automation Control Co., Ltd., Koyo Electronics Industries Co., Ltd. and Shanghai QiYi Electrical & Mechanical Equipment Co., Ltd., respectively. When the angle measuring device is the angular displacement sensor, the model can be WDD35D4A, WDD35D4, M6500 or SX35D4W. Its manufacturer is Zhejiang Huiren Electronic Co.,Ltd., Shanghai Lanbang Electric Appliance Co., Ltd., Shenzhen Chengxin Electronic Technology Co., Ltd. and Shanghai Simi Electronics Co., Ltd. respectively.

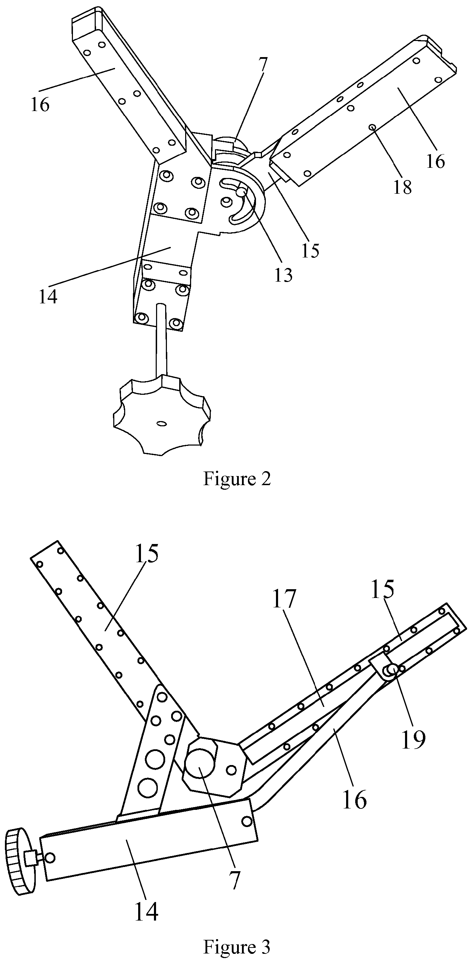

To facilitate the adjustment of the overall structure and the length of the training protector, on the training protector the first lock switch and the length adjusting mechanism of the protector are set. Specifically, the first lock switch is a plug between the end of the mounting plate and the angle adjustment device used for locking the mounting plate and the angle adjustment device. When the plug is inserted into the mounting plate and the locking hole of the angle adjustment device, the mounting plate and the angle adjustment device will be connected. The two will rotate together. After the plug is pulled out, the mounting plate can turn alone, which makes the angle adjustment more flexible and the adjusting range wider. The length adjusting mechanism of the protector includes two kinds of structures. The first one is made up of the slide and the second lock switch. The other one consists of the main location hole, the accessory location hole and the positioning pin. In the actual production they can be flexibly chosen. This technology has no limitation for them. In the first solution, as shown in Figure 3, the slide is set on the mounting plate. The second lock switch is fixed on the supporting plate, which can slide on the slide rail. The positioning on the slide can be achieved by the second lock switch, which is the positioning of the mounting plate and the supporting plate, so as to change the length of the whole structure after connection of the mounting plate and the supporting plate. In the second solution, as shown in Figure 2, at least two main location holes are set on the mounting plate, and at least two accessory location holes are set on the supporting plate. After inserted into the accessory location hole, the locating pin is inserted into different main location holes, so as to change the length of the whole structure after connection of the mounting plate and the supporting plate. So, it can fully adapt to the demand of joint length on different parts of the human body. On one hand, it can improve the wearing comfort. On the other hand it can ensure the effect of the rehabilitation training.

The physiological parameter detecting device is used to collect various physical parameters of the patient, including the temperature sensor, the blood pressure measuring cuff, the pressure sensor, the electrode slice and the ultrasonic probe respectively set on the training protector. Among them, the temperature sensor collects the patient's body temperature data, the blood pressure measuring cuff collects the patient's blood pressure data, the pressure sensor collects the pressure between the patient and the training protector and the current heart rate data, the electrode slice collects the patient's ECG signal, and the ultrasonic probe collects the patient's structure of subcutaneous tissue and blood flow in the vessels.

Further, the control terminal includes the central processing unit, the wireless communication module connected to the central processing unit, and the display and the speaker connected to the central processing unit. Among them, the wireless communication module is connected to the central processing unit. The wireless communication module uses the existing technology. It is any equipment which can receive the information transmitted by the terminal and stored (such as SIM card in the mobile phone). In this implementation example, the wireless communication module is connected to the telemedicine terminal through the wireless communication. The central processing unit also uses the existing technology. It is same as the general central controller. And it sets the body temperature monitoring module, the blood pressure monitoring module, the pressure monitoring module, the ECG monitoring module, the heart rate monitoring module, the subcutaneous tissue structure monitoring module, the vascular blood flow monitoring module, the clock module in it. The body temperature monitoring module, the blood pressure monitoring module, the pressure monitoring module, the ECG monitoring module, the heart rate monitoring module, the subcutaneous tissue structure monitoring module and the vascular blood flow monitoring module are connected to the temperature sensor, the blood pressure measuring cuff, the pressure sensor, the electrode slice and the ultrasonic probe respectively, so that the central processing unit receives and processes the information from the temperature sensor, the blood pressure measuring cuff, the pressure sensor, the electrode slice and the ultrasonic probe. The clock module has the function of timing. All of the above modules use the existing mature technologies. The central processing unit is connected to the angle measuring device to receive the information about the current training angle delivered by the angle measuring device.

The implementation method of this invention is as follows:
The patient fixes the training protector onto the joint to be trained. And then the doctor develops at least a set of training plan according to the actual situation of the patient, including the training angle, the duration of the training and other information. And then it is sent through the telemedicine terminal to the wireless communication module. The wireless communication module receives and stores the plan, and then sends a set of training plan to the central processing unit for processing. Through the monitor, the training angle of the training plan is displayed. The patient manually adjusts the angle adjustment device according to the angle information in the training plan on the monitor, so that a training angle is formed between the upper and lower bracket. At the same time the connection position of the supporting plate on the mounting plate is adjusted, in order to satisfy the demand of the length of corresponding joint of the human body. In the process of adjustment of the training angle of the training protector, the angle measurement device measures the angle in real time, and then transmits the information to the central processing unit for processing and displaying on the monitor. If the angle displayed is not the planned training angle, the patient can adjust the angle of the training protector, such as pulling out the plug and adjusting the inclination of the mounting plate alone until the current training angle is consistent with the angle of the training plan. It's worth noting that to further allow the patient to see the current training angle. On the angle adjustment device of the training protector a mechanical angle dial is set. So, when the training protector adjusts the angle, the patient can directly see the training angle of the current training protector through the mechanical dial.

When the training angle of the training protector is consistent with the angle of the training plan, the patient presses down the timing button set on the central processing unit. The speaker will make the timing noise. At the same time the monitor displays "training started", so as to remind the patient of starting of the training. In the process of training, the patient's joint keeps the angle of the training protector, until the duration of the training reaches that in the training plan. At this point, the central processing unit controls the speaker, which will make the timing end noise. At the same time "training ended" is displayed on the monitor to prompt the end of the training. The central processing unit stops timing, and delivers the training results to the telemedicine terminal through the wireless communication module, so as to provide reference for physicians to make new training plan. The patient adjusts the angle adjustment device, so as to remove the training protector, or adjusts the training angle of the training protector according to the training plan, and then continues the rehabilitation training with another angle.

In the process of training, because the patient's various physiological conditions change, such as rise or fall of body temperature and blood pressure, accelerating of heart rate and blood vessels, etc., the changes of theses physical conditions are likely to make the patient feel uncomfortable or even cause shock, which are very bad for the traction rehabilitation training. Therefore, the physiological parameter detecting device in this invention can collect the patient's physiological parameters in real time, and transmit them to the central processing unit, which receives the physiological parameter information for processing. There are two kinds of processing methods. First, the central processing unit evaluates the current state according to the patient's physiological parameters, adjusts the current training plan, and displays the adjusted training angle on the monitor. The patient adjusts the training angle of the training protector according to the training plan in real time, so that the physiological parameter values all maintain within the normal range; second, they are transmitted to the telemedicine terminal through the wireless communication module. The doctor adjusts the currently implemented training plan according to the patient's current physical parameters, and then transmits to the central processing unit, which controls the monitor displaying the adjusted training plan. And then the patient can adjust the training angle of the training protector according to training plan in real time. With the second method, the doctor adjusts the training plan. They can know the physical conditions of the patient at any time, so as to make e safer and more targeted training plan for the patient's current training situation. The patient can receive better traction rehabilitation training.

At the same time, through the patient's active flexion and extension of the joint, the rotation of the mounting plate is driven. The angle measuring device detects the angle value and transmits to the telemedicine terminal. The telemedicine terminal can also evaluate the patient's joint range of motion according to the physiological parameters of the patient and the rotation angle of the mounting plate, so as to determine the rehabilitation of the patient's joint, and make the next round of rehabilitation training plan correspondingly, so as to improve the effect of training and promote the patient's early recovery.

According to the above implementation example, we can well realize this invention. It's worth noting that based on the above structure design, in order to solve the same technical problem, even some little changes or polishing are made for this invention, and the essence of the technical proposal adopted is still consistent with that of this invention, they also should be within the scope of protection of this invention.

## Claims

1. A system comprising: a training protector (6) and an angle adjustment device set on the training protector (6), as well as a telemedicine terminal (1), a control terminal with wireless connection to the telemedicine terminal (1), and a angle measuring device set on the angle adjustment device (7) used for measuring a training angle of the training protector (6);
the control terminal includes the central processing unit (3) connected to the angle measuring device (7), a wireless communication module connected to a central processing unit (3) with wireless communication (2) with the telemedicine terminal (1), and a display (4) and a speaker (5) respectively connected to a central processing unit And on the training protector (6), a physiological parameter detecting device connected to a central processing unit (3) is set.

2. A system of claim 1, wherein: the angle measuring device (7) is a absolute value encoder or an angular displacement sensor.

3. A system of claim 2, wherein: a physiological parameter detecting device includes a temperature sensor (8), a blood pressure measuring cuff (9), a pressure sensor, an electrode slice (10) and a ultrasonic probe (12) respectively set on the training protector (6) connected to a central processing unit (3).

4. A method of using the joint rehabilitation training system based on the remote control, comprising: the following steps:
(1) the patient fixes a training protector on a joint to be trained;
(2) the telemedicine terminal makes an initial training plan, and sends it to the control terminal;
(3) according to the training plan, a patient uses the angle adjustment device to adjust the training angle of the training protector, so that it is consistent with a training plan;
(4) the training protector is started and a patient begins a rehabilitation training;
(5) during the training, the patient's current physical data is detected with a physiological parameter detecting device and transmitted to a control terminal or a telemedicine terminal, a human body's physiological load and joint/bone tolerance in the training process are monitored in real time, it automatically prompts whether a training intensity is moderate, and ensures the training safety;
(6) after completion of a rehabilitation training, through a physiological parameter detecting device a patient's current physical data is detected and transmitted to the control terminal or a telemedicine terminal, to obtain the new training plan;
(7) step (3)~(6) are repeated until the patient recovers fully.

5. A method of claim 4, wherein: it has the following characteristic, step (3) includes the following steps:
(3a) the wireless communication module receives a training plan and transmits it to a central processing unit;
(3b) the control display of a central processing unit displays a content of the training plan;
(3c) the patient adjusts a training angle of a training protector according to a angle displayed using a angle adjustment device; (3d) the angle measuring device measures the training angle of a training protector in real time and feedbacks to a central processing unit;
(3e) the control display of the central processing unit displays the current angle of a training protector, a patient modifies the angle of the training protector according to the current angle of the training protector displayed on a monitor, so that it is consistent with an angle set in the training plan.

6. A method of claim 5, wherein: in step (3a), a wireless communication module transmits the training plan to a central processing unit and automatically stores.

7. A method of claim 6, wherein: in step (4), when a patient begins the rehabilitation training, a physiological parameter detecting device also begins to collect the physiological parameters of the patient, and transmits to a telemedicine terminal through a wireless communication module, the telemedicine terminal diagnoses the current state of a rehabilitation training of the patient in real time.

8. A method of claim 7, wherein: in step (2), an initial training plan is made according to the physiological state and joint state before a patient begins the first rehabilitation training.
